(19) Europäisches Patentamt
European Patent Office
Office européen des brevets

(11) EP 1 251 122 A1

(12) **EUROPEAN PATENT APPLICATION**

(43) Date of publication:
**23.10.2002 Bulletin 2002/43**

(51) Int Cl.$^7$: **C07D 201/08**

(21) Application number: **01201356.1**

(22) Date of filing: **17.04.2001**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **DSM N.V.**
**6411 TE Heerlen (NL)**

(72) Inventors:
- **Smits, Hubertus Adrianus**
  **6212 GC Maastricht (NL)**
- **Sielcken, Otto**
  **6136 BG Sittard (NL)**
- **Haasen, Nicolaas Franciscus**
  **6141 MB Limbricht (NL)**
- **Guit, Rudolf Philippus Maria**
  **6228 GP Maastricht (NL)**
- **Tinge, Johan Thomas**
  **6137 LL Sittard (NL)**

(54) **Process for the preparation of epsilon-Caprolactam**

(57) The invention relates to a process for the preparation of ε-caprolactam, wherein ε-caprolactam is prepared starting from butadiene, carbon monoxide, hydrogen and ammonia and the process comprises:

1. carbonylating butadiene in the presence of an alkanol and a catalyst comprising palladium, a multidentate phosphine ligand and an acidic co-catalyst to produce alkyl-4-, alkyl-3- and alkyl-2-pentenoate,
1'. optionally isomerising the alkyl-3- and/or alkyl-2-pentenoate into alkyl-4-pentenoate,
2. hydroformylating the alkyl-4-, alkyl-3- and alkyl-2-pentenoate in the presence of a catalyst comprising rhodium and an organic phosphorous containing ligand to produce alkyl-5-formylvalerate,
3. reductively aminating alkyl-5-formylvalerate in the presence of a hydrogenation catalyst comprising ruthenium on a carrier catalyst to produce ε-caprolactam and ε-caprolactam precursors,
4. optionally converting ε-caprolactam precursors at elevated temperature into ε-caprolactam.

EP 1 251 122 A1

## Description

The invention relates to a process for the preparation of ε-caprolactam.

The process which is commonly used in practice for the preparation of ε-caprolactam involves oxidation of cyclohexane to cyclohexanone, reaction with hydroxylamine sulphate or hydroxylamine phosphate to cyclohexanone oxime, and sulphuric acid catalyzed rearrangement of the oxime to ε-caprolactam in oleum (Beckmann rearrangement). In this process the ε-caprolactam is recovered from the reaction mixture by neutralizing with ammonia water and then separating the ε-caprolactam from the ammonium sulphate-containing solution obtained.

A disadvantage of this process is that large amounts of ammonium sulphate by-product are prepared requiring additional investment to recover that by-product. Since ammonium sulphate is considered to be an undesired by-product, efforts have been made to find ways of preparing ε-caprolactam without by-production of ammonium sulphate.

The object of the invention is to provide a process for the preparation of ε-caprolactam in which no ammonium sulphate is produced.

This object is achieved in that ε-caprolactam is prepared starting from butadiene, carbon monoxide, hydrogen and ammonia and the process comprises:

1. carbonylating butadiene in the presence of an alkanol and a catalyst comprising palladium, a multidentate phosphine ligand and an acidic co-catalyst to produce alkyl-4-, alkyl-3- and alkyl-2-pentenoate,

1'. optionally isomerising the alkyl-3- and/or alkyl-2-pentenoate into alkyl-4-pentenoate,

2. hydroformylating the alkyl-4-, alkyl-3- and alkyl-2-pentenoate in the presence of a catalyst comprising rhodium and an organic phosphorous containing ligand to produce alkyl-5-formylvalerate,

3. reductively aminating alkyl-5-formylvalerate in the presence of a hydrogenation catalyst comprising ruthenium on a carrier to produce ε-caprolactam and ε-caprolactam precursors,

4. optionally converting ε-caprolactam precursors at elevated temperature into ε-caprolactam.

It has been found that with the process according to the invention no ammonium sulphate by-product is produced. An advantage of the process of the invention is that the starting compound (butadiene) is less expensive than the starting compound cyclohexane of the process commonly used in practice. Carbon monoxide, hydrogen and ammonia are also cheap and readily available in large quantities. Another advantage of the process according to the invention are the lower investment and production costs. Still another advantage of the process according to the invention is a considerable reduction of emissions of nitrogen oxides and/or sulphur oxides. Another advantage of the process of the invention is that the energy consumption per ton of produced ε-caprolactam is lower compared with the process commonly used in practice. Yet another advantage is that the ε-caprolactam precursors obtained in step 3 can directly be converted to nylon-6.

Several attempts have been made in the prior art for preparing ε-caprolactam from butadiene. Although some of the disclosed processes were successful in preparing ε-caprolactam from butadiene, the overall conversion degree is less than in the process of the invention and/or the applied catalyst systems in one or more of the disclosed process steps do not possess sufficient stability and/or are not economically attractive. With the process of the invention (steps 1, 2, 3 and 4), the overall conversion degree from butadiene to ε-caprolactam is higher than 70%.

The carbonylation stage or step of the process of the invention involves converting butadiene to alkyl-3-pentenoate in one reaction step. As used herein, the term "carbonylation" means every reaction between an unsaturated substrate, carbon monoxide and an alcohol, whereby an ester compound is obtained.

Several methods for preparing alkyl-3-pentenoate starting from butadiene have been described in the prior art. For example, carbonylation processes of butadiene using a cobalt/pyridine catalyst system are known. These processes have the disadvantages of the necessity of the use of high pressures (30-120 MPa) and the use of large amounts of pyridine. Carbonylation processes of butadiene using catalyst systems comprising rhodium, iridium or nickel and an iodide containing promoter or a catalyst system comprising palladium and crotyl chloride are also known in the art. These processes are disadvantageous because of the necessity of the presence of considerable to stoichiometric amounts of halides resulting in corrosion of equipment and hence in the use of expensive types of construction material for the reaction installations. Another disadvantage of the use of a catalyst system rhodium, iridium or nickel and an iodide containing promoter is that the carbonylation product contains organo-iodide impurities which need to be separated.

The carbonylation is performed in the presence of a carbonylation catalyst comprising palladium, a multidentate phosphine ligand and an acidic co-catalyst such as for example described in US-A-6010975, WO-A-9506027, EP-A-273489, WO-A-9845040, US-A-5495041 and WO-A-0056695; the disclosures of which are incorporated herein by reference. A preferred carbonylation process useful in this invention is described in WO-A-0056695. The specific catalyst system described herein results in high catalyst activity, which allow for molar ratios well over 200:1 and suitably well over 300:1 of conjugated diene to palladium to be used, whilst still obtaining high selectivities to the alkyl pentenoate

compounds.

The multidentate phosphine ligand is preferably a bidentate phosphine ligand. A preferred class of bidentate phosphine ligands is for example described in EP-A-273489, a more preferred class of bidentate phosphine ligands is described in the above mentioned WO-A-0056695.

Examples of these more preferred bidentate phosphine ligands include symmetric or asymmetric [3,3,1] or [4,2,1] isomers of 1,2-P,P'bis(1,5-dimethyl, 9-phosphabicyclononyl)ethane and mixtures thereof or symmetric or asymmetric [3,3,1] or [4,2,1] isomers of 1,3-P,P'bis(1,5-dimethyl, 9-phosphabicyclononyl)propane and mixtures thereof or symmetric or asymmetric [3,3,1] or [4,2,1] isomers of 1,2-P,P'bis (1,5-dimethyl 9-phosphabicyclononyl)propane and mixtures thereof, or a combination of above mentioned diphosphines.

The above mentioned bidentate phosphine ligands can be prepared as for example described in WO-A-0056695.

A wide range of acidic co-catalysts can be used. More preferably a carboxylic acid is used. The carboxylic acid is preferably an organic compound with 1 to 30 carbon atoms. The pKa of the acid is preferably greater than 2 when measured in an aqueous solution of 18°C The pKa is preferably less than 5. Exemplary carboxylic acids are benzoic acid, acetic acid, valeric acid, butanoic acid, or nonanoic acid. Also acids corresponding with the ester (by-) products can be advantageously used in step 1 of the process of the invention. The use of these acids is advantageous because they are readily obtainable by hydrolysis of these ester (by-)products. Examples of these acids are nonadienoic acids, monoalkyladipate, pentenoic acid, 1-butene-2-carboxylic acid and methyl-substituted butenoic acid. The acidic co-catalyst is preferably pentenoic acid. The use of pentenoic acid is advantageous because any reaction of pentenoic acid with the alkanol results in formation of alkyl pentenoate compound.

The palladium may be present in the carbonylation reaction mixture as a heterogeneous palladium compound or as a homogeneous palladium compound. However, homogeneous systems are preferred. Since palladium in situ forms a complex with the multidentate ligand, the choice of the initial palladium compound is in general not critical. Preferably, a salt of carboxylic acid is used, suitably a carboxylic acid with up to 10 carbon atoms, such as palladium acetylacetonate, palladium acetate, palladium nitrate. A very suitable source is palladium(II) acetate.

The multidentate ligand:palladium molar ratio is as a rule between 1 to 10, preferably from 1 to 2. When this ratio is lower than 1 palladium can precipitate, whereas when this ratio is higher than 10, excessive ligand consumption can occur. It has been found that step (1) of the process of the invention is preferably carried out with a slight excess molar excess of ligand to palladium because the ligand degradation is decreased or even no ligand degradation occurs. Most preferably, the multidentate ligand/palladium molar ratio is higher than 1.05 and less than 1.2. When performing step (1) with a slight excess of ligand to palladium, it will be preferred to monitor the concentration (and degradation) of the ligand during the course of the process and add fresh ligand in order to remain in the preferred ranges of operation.

The quantity of the acidic co-catalyst may vary within wide ranges. Preferably, the amount of acid ranges from 0.1 to 40 wt.%, preferably between 0.5 and 10 wt.% and more preferably between 1 to 8 wt.% (based on the total reaction mixture).

The palladium concentration in the reaction mixture is preferably as high as possible because the rate of reaction per unit of reactor volume will be higher. The upper limit for a homogeneous catalyst system will normally be determined by the solubility of palladium in the reaction mixture. The upper limit can be determined by one skilled in the art. Preferably, the amount of palladium is between 400 and 4000 ppm and more preferably between 800 and 1200 ppm.

The process of the present invention can be used for each range of mole conjugated diene per mole of palladium cation. The ratio of mole conjugated diene per mole of palladium cation can vary between wide limits, suitably in the range from $1x10^1$ to $2x10^7$ mole conjugated diene per mole of palladium cation, dependent of a continuous, semi-continuous or batch-wise process is being used.

The butadiene can be used in pure form or in an admixture with aliphatic compounds. For instance, an exemplary of such admixture is the $C_4$-cut obtained in a steam cracker process. The $C_4$-cut can comprise butadiene plus 1-butene, 2-butene, isobutene and butane.

The carbon monoxide can be used in a pure form or diluted with an inert gas such as, for example nitrogen, rare gases or carbon dioxide. Oxygen and sulphur should not be present. Small quantities of hydrogen and/or water may be present. It is therefore possible to use, for example, technical carbon monoxide with 0.1-3 vol.% hydrogen. In general, more than 5% hydrogen is undesirable, since this can cause hydrogenation of butadiene and/or hydroformylation of butadiene under the carbonylation conditions. The amount of carbon monoxide is not critical if at least a stoichiometric amount of carbon monoxide relative to butadiene is supplied to the carbonylation reaction.

Step (1) of the process of the invention should be performed in the absence of any oxygen or should at least be performed in the presence of oxygen as low as possible. The presence of oxygen is disadvantageous because the presence of oxygen may result in palladium and/or ligand losses.

The temperature of the carbonylation is between 25 °C and 200 °C and preferably between 100 °C and 180 °C. The pressure is not particularly critical and generally ranges between 0.5 MPa and 20 MPa, although it is preferably

greater than 1 MPa. A very high pressure is disadvantageous because the process equipment will become very expensive. A practical and preferred upper limit is therefore about 10 MPa.

The alkanol is preferably an aliphatic alcohol with one or more hydroxy groups. By preference, the alkanol has one hydroxy group. Alkanols with formula ROH are useful, in which R is a linear or branched alkyl group with preferably 1 to 6 carbon atoms. Exemplary alkanols are methanol, ethanol, propanol, isopropanol, butanol, tert-butanol, pentanol and hexanol. Most preferably methanol and ethanol and most preferred methanol is used as the alkanol. It has been found that in a continuous process the alkanol fed to step (1) is advantageously a mixture of fresh alkanol and recycled alkanol released in the reductive amination (step (3)) of the process of the invention. The fact that at least a part of the necessary amount of alkanol can be recycled in the process of the invention is advantageous because this results in an even further decrease of the production costs.

All inert solvents are in principle suitable as an additional solvent, but it is also possible to use an excess of one of the reactants or (by-)products in such an amount that a suitable liquid phase is formed. Suitable reactants or by-products are the pentenoate esters, C9-alkyl esters and/or high boiling by-products such as for example diesters. Examples of diesters are C6-diesters, such as for example dialkyladipate, dialkylmethylglutarate, dialkylethylsuccinate and dialkylpropylmalonate. These high boiling by-products are found to be very suitable solvents. Suitable extra inert solvents are for example polar, aprotic compounds, such as ketones, ethers, e.g. diphenylether, anisole, tetrahydrofuran or the dimethylether of diethyleneglycol, or sulphones, e.g. sulpholane.

As a rule, the mixture leaving the carbonylation step contains the following isomeric pentenoates: 0 - 3 % alkyl-4-pentenoate, 90 - 100 alkyl-3-pentenoate and 0 - 10 alkyl-2-pentenoate, in which the total of alkyl pentenoates add up to 100%.

The carbonylation can be performed batch wise, semi-continuously or continuously.

In a preferred embodiment the carbonylation is performed on a continuous or semi-continuous basis. An example of a semi-continuous process for the preparation of pentenoate ester is a process in which a stirred tank reactor is filled with the catalyst system, the solvent and reactants and to which reactor continuously butadiene and optionally the alcohol are continuously supplied. Carbon monoxide is continuously dosed to the reactor or is continuously present as a gaseous mixture.

Preferably a continuous process is used. An example of reactor system for a continuous process is one (well mixed) bubble column reactor or a number of (well mixed) bubble column reactors in series or in parallel in which a catalyst system, a solvent (optional), butadiene, carbon monoxide and alcohol are fed to a first reactor. The resulting reaction mixture in the first reactor is fed to a second reactor. Carbon monoxide and optionally fresh butadiene and alcohol are optionally fed to the second and further reactors in the appropriate amounts. The catalyst system containing reaction mixture leaving the last reactor is preferably subjected to a separation in which carbonylation products and unconverted starting products are separated, the remaining mixture containing catalyst system is returned to the first reactor and/or to the subsequent reactors.

Separating carbon monoxide, butadiene, alcohol and pentenoate ester from the reaction mixture which comprises the catalyst system can be performed in various ways. Generally carbon monoxide is separated first from the reaction mixture in, for example, a simple gas-liquid separation unit. Butadiene, alcohol and pentenoate ester can be separated from the reaction mixture containing the catalyst system in one or more steps followed by isolating pentenoate ester from butadiene and alcohol. Preferably butadiene and alcohol are separated from the reaction mixture in a separate step followed by the isolation of pentenoate ester from the remaining reaction mixture. More preferred, carbon monoxide, butadiene and light compounds are separated from the reaction mixture in a separate step at ambient or elevated temperature up to about 200 °C, preferably no more than 150 °C, and normal or superatmospheric pressure. This separation step is then followed by isolation of catalyst system from the remaining reaction mixture.

The various compounds can be separated using a variety of techniques such as, for example, by simple flash operation, distillation, membrane separation, extraction, adsorption using an ion exchanger. The choice of unit operation is a function of the physical properties of the compounds to be separated.

The remaining mixture containing catalyst system comprising ligand, Pd and carboxylic acid and, for example, high boiling by-products and a solvent if present, are returned to the reaction zone to be used in a further carbonylation. In order to prevent a build up of, for example, high boiling by-products in this circulating reaction mixture, a part of this mixture may be purged and reprocessed to retrieve, for example, palladium and/or phosphine ligand.

In a subsequent step (1'), which is optionally and preferably omitted in the process of the invention, the carbonylation reaction mixture (containing alkyl-4-, alkyl-3- and alkyl-2-pentenoate) is subjected to an isomerisation reaction. The isomerisation reaction involves converting the alkyl-3-pentenoate (and optionally the alkyl-2-pentenoate) to alkyl-4-pentenoate in the presence of an isomerisation catalyst. Suitable isomerisation catalysts and isomerisation conditions are for example described in US-A-4801738 and WO-A-9426688, the complete disclosures of which are incorporated as reference.

It may be advantageous to enrich the mixture obtained in the isomerisation step with respect to the alkyl-4-pentenoate. Suitable separation techniques are distillation, crystallization and absorption. After being liberated of

the alkyl-4-pentenoate, the remaining mixture, substantially consisting of alkyl-3-and 2-pentenoate, is preferably recycled to the isomerisation step.

In a subsequent step (2), the mixture leaving the carbonylation step or isomerisation step (containing alkyl-4-, alkyl-3-pentenoate and alkyl-2-pentenoate), is subjected to a hydroformylation reaction in the presence of a catalyst comprising rhodium and an organic phosphorus containing ligand to produce alkyl-5-formylvalerate. In case the isomerisation step (1) is carried out, preferred organic phosphorous containing ligands are monodentate organic phosphines as for example described in US-A-4801738 and WO-A-9426688.

Several methods for preparing alkyl-5-formylvalerate from alkyl-4- and alkyl-3-pentenoate and optionally alkyl-2-pentenoate have been described in the prior art. For example, hydroformylation processes of pentenoate isomers using a catalyst system of platinum, a bidentate phosphine and an acidic co-catalyst are known. These processes have the disadvantages of the necessity of the use of high pressures, instability of the catalyst system and a low selectivity. Hydroformylation processes using cobalt containing catalyst systems are also known in the prior art. These processes are disadvantageous in that they require high pressures and result in low selectivities. Hydroformylation using ruthenium containing catalyst systems have the disadvantage of a low selectivity, mainly due to the hydrogenation of the pentenoate isomers into valerate compounds.

Preferably, before introducing the mixture leaving the carbonylation step or isomerisation step in the hydroformylation step, this mixture is substantially freed of hydroperoxide compounds as for example described in EP-A-662468. The catalyst activity is improved and the catalyst remains stable for a longer period of time when the alkyl pentenoate mixture is freed from hydroperoxide compounds. This is because the hydroperoxide compounds have proved to be catalyst poisons inasmuch as these compounds readily convert the phosphorous ligands into catalytically inactive compounds. Suitable methods for removing hydroperoxide compounds are for example described in EP-A-662468. A preferred method for removing hydroperoxide compounds is passing the pentenoate mixture over ordinary activated (dry) alumina at room temperature or slightly elevated temperature. The contacting with alumina can advantageously be performed in a continuous process. The alumina is then preferably placed in a packed bed reactor. The mixture which is introduced in the hydroformylation step is preferably substantially freed of (strong) acidic compounds with a method known to a man skilled in the art. For example, strong acidic compounds can be removed by contacting the mixture with a basic compound, for example a basic resin. An example of such a basic resin is a packed bed of polystyrene-divinylbenzene matrix containing basic amine groups. The resin is preferably placed in a packed bed reactor.

Step (2) of the process of the invention should be performed in the absence of any water and/or oxygen or should at least be performed in the presence of oxygen and/or water as low as possible. The presence of oxygen and/or of water is disadvantageous because the presence of oxygen and/or water may result in rhodium and/or ligand losses.

In a preferred embodiment of the invention, the isomerisation step (1) is omitted and the mixture leaving the carbonylation stage (containing alkyl-4-, alkyl-3- and alkyl-2-pentenoate) is subjected to a hydroformylation reaction in the presence of a catalyst comprising rhodium and a multidentate organic phosphite ligand. Suitable multidentate phosphite ligands are for example disclosed in WO-A-9518089 and WO-A-9733854, the disclosures of which are incorporated herein by reference. Hydroformylation of the carbonylation mixture using a rhodium/phosphine or monodentate phosphite has the disadvantage of a low selectivity to terminal aldehydes. A preferred hydroformylation process useful in this embodiment of the invention is described in WO-A-9518089 and WO-A-9733854. A more preferred hydroformylation process useful in this invention is disclosed in WO-A-9733854. The multidentate phosphite ligand has the following general formula

$$A - \left[ O - P \begin{matrix} / (OR^1) \\ \backslash (OR^2) \end{matrix} \right]_n$$

where $R^1$ and $R^2$ are the same or different monovalent aromatic organic groups which are not connected to each other in any way other than via the phosphorous atom P and where A is an n-valent group or atom and where n is an integer of at least 2 and where the respective [-O-P($OR^1$)($OR^2$)] group may be the same group or different groups and where the phosphite forms a chelate-type complex with rhodium.

The structure of the bridging group A of the multidentate phosphite ligand is chosen such that the multidentate phosphite ligand can form a chelate-type complex with rhodium. By a chelate-type complex is meant that (substantially) at least two phosphorous atoms of a ligand molecule form a coordinated bond with one rhodium atom/ion. By a non-chelate type complex is meant that only one phosphorus atom of a ligand molecule forms a coordinated bond with one rhodium atom/ion. The choice of the bridging group of the ligand will determine whether a chelate-type complex can be formed in the reaction zone. Examples of bridging groups which result in a ligand which can form a chelate-type complex are for example described in WO-A-9518089. EP-A-518241 describes a process for preparing aldehydes by hydroformylation of olefinically unsaturated organic compounds, for example 1-octene or dimerised butene, in which a bidentate organic phosphite ligand is used. A disadvantage of the process according to EP-A-518241 is that the selectivity to terminal aldehydes when starting from ethylenically internally unsaturated functionalised organic compounds (like alkyl-3- and alkyl-2- pentenoate compounds) is generally too low for a commercially attractive process. However with some of the disclosed multidentate phosphites of EP-A-518241, such as tetrakis(di-(2,4-di-tert-butylphenyl)phosphito)-pentaerythritol, reasonable selectivities to terminal aldehydes are achieved. A drawback of the use of these "high selectivity" ligands is that the hydroformylation activity of the catalyst system is too low for a commercially attractive process. Increasing the catalyst activity by increasing the temperature is not possible because the ligands are thermally unstable at higher temperatures. In addition, selectivity decreases at higher temperature, because the rate of competing hydrogenation reactions increases with temperature more rapidly than the rate of the hydroformylation reaction. A preferred bridging group is a 2,2'-dihydroxyl-1,1'-binaphtalene bridging group with the following structure

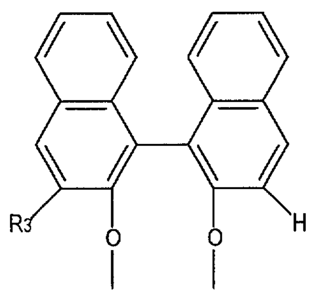

or

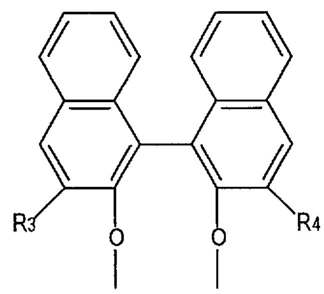

which $R^3$ and $R^4$ are substituents other than hydrogen. The use of such a bridging group results in high selectivities to the terminal alkyl-5-formylvalerate, combined with a relatively high catalyst activity.

The substituents $R^3$ and $R^4$ are preferably organic groups containing at least one carbon atom, and more preferably containing 1-20 carbon atoms.

Preferably $R^3$ and $R^4$ are individually selected from the group of alkyl, aryl, triarylsilyl, trialkylsilyl, carboalkoxy, carboaryloxy, aryloxy, alkoxy, alkylcarbonyl, arylcorbonyl, oxazole, amide, amine or a nitrile.

For $R^3$ and $R^4$, the alkyl group is preferably a $C_1$-$C_{10}$ alkyl group, for example methyl, ethyl, propyl, isopropyl, butyl, tert-butyl, isobutyl, pentyl or hexyl. An example of a suitable triarylsilyl group is triphenylsilyl and examples of a suitable trialkylsilyl group are trimethylsilyl and triethylsilyl. Preferred aryl groups have 6 to 20 carbon atoms, for example phenyl, benzyl, tolyl, naphthyl, anthranyl or phenanthryl. Preferred aryloxy groups have 6 to 12 carbon atoms, for example phenoxy. Preferred alkoxy groups have 1 to 20 carbon atoms, for example methoxy, ethoxy, tert-butoxy or isopropoxy. Preferred alkylcarbonylgroups have 2 to 12 carbon atoms, for example methylcarbonyl, tert-butylcarbonyl. Preferred arylcarbonyl groups have 7 to 13 carbon atoms, for example phenylcarbonyl. Preferred amide groups contain a $C_1$-$C_4$ alkyl group and preferred amide groups contain two $C_1$-$C_5$ alkyl groups.

Most preferably, $R^3$ and $R^4$ are individually a carboalkoxyl or a carboaryloxy group, $-CO_2R$, in which R is a $C_1$-$C_{20}$ alkyl group or a $C_6$-$C_{12}$ aryl group and preferably a $C_1$-$C_8$ alkyl group. Examples of suitable R-groups are methyl, ethyl, propyl, isopropyl, n-butyl, tert-butyl, isobutyl, phenyl and tolyl. Even more preferably both $R^3$ and $R^4$ are the same carboaryloxy and more preferably the same carboalkoxyl group because the resulting ligands are more easily obtainable.

The 2,2'-dihydroxyl-1,1'-binaphthalene bridging group can optionally be further substituted with other groups, for example halogen, for example Cl or F or one of the substituents which may be present on the bridging group as described above. It has been found that when using these ligand compounds a high selectivity to alkyl-5-formylvalerate can be achieved at a high reaction rate. These ligands may be prepared by a variety of methods known in the art, for example, see descriptions in US-A-4769498, US-A-4688651 and *J. Amer. Chem. Soc.*, **1993,** 115, 2066. The bidentate phosphite compounds according to the invention can be prepared with the 3- or 3,3'-substituted 2,2'-dihydroxy-1,1'-binaphthalene bridging compounds. The binaphthol bridging compounds can be prepared by procedures as described

in *Tetrahedron Letter,* **1990,** 31(3), 413-416 or in *J. Amer. Chem. Soc.,* **1954,** 76, 296 and *Org. Proc. Prep. International,* **1991,** 23, 200. The phosphite compounds can be prepared by using the process as described in US-A-5235113 to couple these binaphthol bridging compounds with phoshoro-chloridites, $(R^1O)(R^2O)PCl$, prepared by treating $R^1OH$ and/or $R^2OH$ with $PCl_3$.

The multidentate phosphite ligand is preferably a bidentate phosphite ligand (n=2).

In the preferred embodiment of the process of the invention (in which the isomerisation step is omitted), the hydroformylation reaction is preferably performed in the presence of an acid compound having a $pK_a$ between 1 and 12 when measured in water of 18 °C as for example described in WO-A-9819990. It has been found that the catalyst activity can further be stabilized over a prolonged period of time when such an acid is present. The acid is preferably present in an amount of 0.05 to 20 wt.% during the hydroformylation reaction. More preferably between 0.1 and 1 wt. %. The acid can be any acid with a $pK_a$ between 1 and 12 and preferably between 2.5 and 10, measured in water of 18°C. Examples of suitable acids are aromatic carboxylic acids, for example optionally substituted benzoic acid, p-chloro-benzoic acid, phthalic acid, aliphatic carboxylic acids, for example dicarboxylic acids having between 2-20 carbon atoms, for example adipic acid, glutaric acid and fumaric acid, mono carboxylic acids, for example valeric acid, butynic acid, decanoic acid, mono methyl adipate, mono methyl glutarate, phenols, for example phenol, cresol, p-methylphenol, bisphenols, bis-β-naphthol, dihydroxy naphthalene. A preferred acid is mono methyl adipate because this acid is readily available and is intrinsically present in step (2) of the process of the invention. Preferably the acid has a normal boiling point higher than 200°C.

The catalyst system used in the hydroformylation step of the process according to this invention can be prepared by mixing a suitable rhodium compound with the phosphite ligand, optionally in a suitable solvent, in accordance with well-known complex-forming methods. The solvent will generally be the solvent used in the hydroformylation. Suitable rhodium compounds are for example hydrides, halides, organic acid salts, acetylacetonates, inorganic acid salts, oxides, carbonyl compounds and amine compounds of these metals. Examples of suitable catalyst precursors are, for example, $RhCl_3$,$Rh(NO_3)_3$, $Rh(OAc)_3$, $Rh_2O_3$, $Rh(acac)(CO)_2$, $Rh(CO)_2(DPM)$, $[Rh(OAc)(COD)]_2$, $Rh_4(CO)_{12}$, $Rh_6(CO)_{16}$, $RhH(CO)(Ph_3P)_3$, $[Rh(OAc)(CO)_2]_2$, and $[RhCl(COD)]_2$, (wherein "acac" is an acetylacetonate group; "Ac" is an acetyl group; "COD" is 1,5-cyclooctadiene; and "Ph" is a phenyl group, DPM is 2,2,6,6-tetramethyl-3,5-heptanedionate group). However, it should be noted that the rhodium compound is not necessarily limited to the above listed compounds.

In order to further improve the stability of the catalyst system it is preferred to add some organophosphorous compound which has a coordination strength to rhodium less than the multidentate phosphite ligand as for example described in WO-A-9819984. Examples of suitable organophosphorous compounds are phosphites and monodentate phosphines. The presence of the organophosphorous compounds avoids oxidation of the phosphite ligand and/or keeps rhodium in solution in case the amount of multidentate phosphite ligand becomes less than stoichiometric (relative to rhodium).

Preferred phosphine compounds have a steric parameter θ of between 160°-220°. This preferred monodentate phosphine can be represented by the general formula $P(R')_3$, where the R' groups are organic groups that are chosen so that the steric parameter θ of the phosphine is between 160° and 220°, preferably between 170° and 210°. The steric parameter θ is the top angle of a cylindrical cone, centred at 2.28 Å ($10^{-10}$ m) from the centre of the phosphorus atom, which just touches the Van der Waals radii of the outer atoms of the R' substituents of a symmetrical $P(R')_3$-phosphine (see also "Chemical Reviews, 1977, Volume 77, pp. 313-348" by C.A. Tolman and US-A-4169861).

The organic group R' of the $PR'_3$ phosphine is preferably an aliphatic, alicyclic or aromatic group with 1-20 carbon atoms, preferably 5-12 carbon atoms, and the three R' groups may be the same or different. The R' group may contain one or more hetero atoms, for example oxygen, nitrogen or a halogen.

Examples of monodentate phosphines according to the invention are tri(isopropylphosphine), tri(sec-butyl) phosphine, tribenzylphosphine, tricyclohexylphosphine, dicyclohexylphenylphosphine, di(t-butyl)phenylphosphine, trineopentylphosphine, tri(t-butyl)phosphine, tri-(o-hydroxyphenyl)phosphine, tri(o-methoxyphenyl)phosphine, tri(pentafluorophenyl)phosphine, tri(o-tolyl)phosphine and trimesityl-phosphine. A mixture of two or more of these compounds is also suitable for use as the monodentate phosphine. Preferably, the $PR'_3$ phosphine is trineopentylphosphine, tri(t-butyl)phosphine or tri(o-tolyl)phosphine.

Most preferably, tri(o-tolyl)phosphine is used as the monodentate phosphine in the process according to the invention. Tri(o-tolyl)phosphine is cheap, readily obtainable and shows a high effectiveness in small amounts.

Examples of stabilizing phosphite compounds are triphenylphosphite, tri(p-tolyl)phosphite, tri(isopropyl)phosphite, tri(o-tolyl)phosphite, tri(o-isopropylphenyl)phosphite, tri(t-butyl)phosphite, tri(o-t-butylphenyl)phosphite, tri (2,6-dimethylphenyl) phosphite, tri(2,4-di-t-butylphenyl)phosphite, penta-erythyl-(2,4-di-t-butylphenylphosphite) and the commercially available Ultranox and Weston phosphite compounds of General Electric Plastics. Examples of preferred stabilizing phosphite compounds are tri(o-t-butylphenyl)phosphite, tri(2,6-dimethylphenyl)phosphite and tris (2,4-di-t-butylphenyl)phosphite.

The hydroformylation process can be performed as described below. The temperature is preferably between

room temperature and 200°C, and more preferably from about 50 to 150°C. The pressure may vary from atmospheric pressure (0.1 MPa) to 20 MPa, preferably from 0.15 to 10 MPa and more preferably from 0.2 to 1 MPa. The pressure is generally equal to the combined hydrogen and carbon monoxide partial pressure. Extra inert gasses may however be present. The molar ratio hydrogen : carbon monoxide is generally between 10:1 and 1:10 and preferably between 6:1 and 1:2.

The amount of rhodium (compound) is not specially limited, but is optionally selected so that favorable results can be obtained with respect to catalyst activity and process economics. In general, the concentration of rhodium or iridium in the reaction medium is between 10 and 10,000 ppm and more preferably between 50-1000 ppm, calculated as free metal.

The molar ratio of multidentate phosphite ligand to rhodium is generally from about 0.5 to 100 and preferably from 1 to 10 (mol ligand/mol metal) and most preferably less than 1.2. Preferably the ratio is higher than 1.05. Small deviations in ligand or rhodium concentration will then not automatically result in a lower yield to alkyl 5-formylvalerate. It has been found that by performing the process with such a slight molar excess of ligand to rhodium the ligand degradation, due to other causes than oxidation, is decreased. When performing the process with a slight excess of ligand to rhodium it will be preferred to monitor the concentration (and degradation) of the ligand during the course of the continuous process and add fresh ligand in order to remain in the preferred ranges of operation.

The choice of solvent is not critical provided the solvent is not detrimental to catalyst, reactant and/or product. The solvent may be the mixture of reactants, such as the starting unsaturated compound, the aldehyde product and/or by-products. Suitable solvents include saturated hydrocarbons, for example kerosene, mineral oil or cyclohexane, ethers, for example diphenyl ether, tetrahydrofuran or a polyethyleneglycol, for example Carbowax TM-400, ketones, for example methyl ethyl ketone or cyclohexanone, nitriles, for example 2-methylglutaronitrile or benzonitrile, aromatics, for example toluene, benzene or xylene, esters, for example methyl valerate or caprolactone, Texanol® (Union Carbide), or dimethylformamide, sulfones (for example tetramethylenesulfone).

The hydroformylation can be performed in any kind of reactor which enables the reactants to be well mixed with the catalyst system and carbon monoxide and hydrogen.

As a rule, the mixture leaving the hydroformylation step contains the following linear and branched alkyl formyl valerates: 87-100 % alkyl-5-formylvalerate, 0 - 6 % alkyl-4-formylvalerate, 0 - 4 % alkyl-3-formylvalerate and 0 - 3 % alkyl-2-formylvalerate, in which the total of alkyl formylvalerates add up to 100%.

The hydroformylation can be performed batch wise, semi-continuously or continuously. Preferably, the hydroformylation is performed on a continuous or semi-continuous basis.

The continuous process is preferably performed by continuously removing part of the liquid reaction medium from the hydroformylation reactor (reaction section). This mixture comprises the linear alkyl 5-formylvalerate and branched alkyl formylvalerate compounds, the catalyst system, optionally the solvent, by-products, unreacted alkyl pentenoate and carbon monoxide and hydrogen dissolved in said medium. In a first step any carbon monoxide and hydrogen is removed from this mixture by reducing the pressure to for example atmospheric pressure in for example a flash operation. This carbon monoxide and hydrogen may be reused in the hydroformylation. The resulting liquid mixture is contacted with a basic compound, for example a basic resin, as described in WO-A-8503702 and EP-A-285136. It has been found that specific acids may form due to degradation of the phosphite ligand in the hydroformylation process. It is believed that traces of these specific acids have a detrimental effect on the stability of the phosphite ligand. It is therefore advantageous to separate these acid compounds to avoid a build up of these acids. Examples of basic compounds are metal oxides, for example CaO and MgO. Preferably heterogenic basic compounds are used and most preferably the basic compound is a resin having basic groups. Examples of possible basic groups are secondary or tertiary amine groups, according to -NHR or $-NR_2$, in which R is an organic group, preferably a $C_1$-$C_6$ alkyl group, more preferably methyl. An example of a suitable resin having basic groups is a packed bed of a polystyrene matrix containing basic amine groups. Commercially available resins which can be used are for example Amberlist A-21, A-22, A-23 and A-26 (Amberlist is a brand name of Rohm & Haas). The alkyl 5-formylvalerate, unreacted alkyl pentenoate and low boiling by-products, for example alkylvalerate and branched aldehydes are preferably separated from the catalyst system and high boiling compounds in one or more distillation operations performed at reduced pressure. Another suitable separation method is membrane separation as for example described in WO-A-9634687. Other methods of performing this separation are of course also possible. The catalyst system and high boiling compounds are recycled to the hydroformylation reactor. Preferably any unreacted alkyl pentenoates are also recycled to the hydroformylation reactor.

The substrate and reaction products (mainly alkyl-2-pentenoate, alkyl-3-pentenoate, alkyl-4-pentenoate, alkyl valerate and most of the aldehyde products) which are separated from the catalyst system are preferably separated from each other by distillation. Other separation techniques, for example extraction and crystallization, are however also possible. Preferably, the product stream which is separated from the catalyst system and high boiling compounds is fed to a vacuum distillation unit in which aldehyde products (alkyl-5-formylvalerate and branched isomers) are separated from this product stream. The alkyl 5-formylvalerate is preferably separated from its branched by-products (alkyl

2-, alkyl 3- and alkyl 4-formylvalerate) by distillation as for example described in WO-A-9706126. These branched products can be burned for energy recovery and/or steam generation or can be decarbonylated to the alkyl pentenoate starting compound by well known processes. The product stream, obtained after having separated aldehyde products, is preferably fed to a distillation unit in which alkyl valerate, alkyl-4-pentenoate and cis-alkyl-2-pentenoate are separated from trans-alkyl-2-pentenoate and alkyl-3-pentenoate. Trans-alkyl-2-pentenoate and alkyl-3-pentenoate are recirculated to the hydroformylation reactor after having been substantially freed of hydroperoxide compounds. A preferred mode of removing hydroperoxide compounds is described in EP-A-662468. The product stream containing alkyl valerate, alkyl-4-pentenoate and cis-alkyl-2-pentenoate can be burned for energy recovery and/or steam generation or can be subjected to hydrogenation for forming alkyl valerate which can be used as solvent.

Most preferably, the trans-alkyl-2-pentenoate /alkyl-3-pentenoate mixture is freed from hydroperoxide compounds by passing this mixture over ordinary activated (dry) alumina at room temperature, causing the hydroperoxide compounds to decompose and/or to remain on the alumina. The contacting with alumina can advantageously be performed in a continuous process. The alumina is then preferably placed in a packed bed reactor.

Preferably, purge flows are present in the process to prevent an accumulation of by-products and the degradation products of the phosphite ligand complex. These purge flows mostly comprise an amount of the rhodium/phosphite catalyst system. The concentration of rhodium in such a purge flow will generally be higher than 100 ppm rhodium and lower than 2000 ppm rhodium. For a commercially interesting process it is necessary to recover the catalyst system comprising the rhodium/phosphite ligand complex from such a purge flow. The rhodium/phosphite ligand complex can advantageously by recovered from such purge flows using a membrane separation process as described in WO-A-9634687.

In a subsequent step (3), the mixture leaving the hydroformylation step (containing mainly alkyl-5—formylvalerate; herein after referred to as alkyl-5-formylvalerate mixture) is subjected to a reductive amination reaction. The reductive amination reaction involves converting the alkyl-5-formylvalerate to ε-caprolactam and ε-caprolactam precursors in the presence of a hydrogenation catalyst comprising ruthenium on a carrier as for example described in WO-A-9835938 or in WO-A-0014062, the disclosures of which are incorporated herein as references. The use of a ruthenium on titanium oxide catalyst is especially advantageous in that this catalyst possesses a high mechanical and chemical stability and/or a high activity. It has been found that the mechanical stability of the catalyst can even further be improved by calcining the titanium oxide carrier at a temperature of between 600 and 700 °C, preferably at a temperature of between 600 and 650 °C. Such a calcination step is especially advantageous in case reactor types are used in which the catalyst is exposed to a considerable amount of mechanical stress which involves danger of attrition.

The reductive amination is performed by contacting the alkyl-5-formylvalerate mixture with the catalyst, hydrogen and an excess of ammonia in a suitable solvent. Suitable solvents are ammonia, water, tertiary amines, ethers, alcohols with 1-6 carbon atoms, ethers with 2 to 10 carbon atoms, for example methyl-tertiary butyl ether and tertiary amylmethyl ether or high boiling organic solvents. Preferably, ammonia, an alcohol and/or water are used as solvent. From the alcohols the one corresponding to the alkyl group of the alkyl-5-formylvalerate is preferred. More preferably, water, water/corresponding alkanol mixture or ammonia is used as solvent. In case ammonia is used as solvent, ammonia has both the function of solvent and reactant. Water will be formed in the reductive amination step as a reaction product of the reaction between the formyl group of the alkyl formylvalerate compound and ammonia. The most preferred solvent is a water/corresponding alkanol mixture because the 5-formylvalerate ester has increased solubility in these mixtures compared to pure water. The water content in the reaction mixture is at least 10 wt.%, more preferably between 15 and 60 wt.% and most preferably between 20 and 50 wt.%. The concentration of the alkanol is preferably between 1 and 15 wt.%.

The reaction mixture obtained in the reductive amination step comprises ε-caprolactam and ε-caprolactam precursors, ammonia, hydrogen, water and the corresponding alkanol. With ε-caprolactam precursors is meant 6-aminocaproic acid, 6-aminocaproate ester, 6-aminocaproamide and/or oligomers of these compounds. If the solvent is water, the ε-caprolactam precursors will mainly consist of 6-aminocaproic acid and 6-aminocaproamide and oligomers thereof. If the solvent is an alcohol and/or ammonia, the ε-caprolactam precursors will mainly consist of 6-aminocaproate ester and oligomers thereof. In case the reductive amination mixture contains 6-aminocaproate ester in an amount higher than 0.5 wt.% (calculated on the amount of ε-caprolactam precursors and side-reaction products, which may be present in small amounts, present in the reductive amination mixture), it is preferred to contact the reductive amination mixture with water in order to hydrolyse the ester group of 6-aminocaproate ester. A preferred method therefore is described in for example WO-A-9942440. Hydrolysing the ester group of 6-aminocaproate ester before introducing the reductive amination mixture in the cyclisation step (which is optionally in the process of the invention and in which the ε-caprolactam precursors are converted to ε-caprolactam) is advantageous because it has been found that performing the cyclisation step in the presence of 6-aminocaproate ester promotes the formation of the corresponding N-alkyl caprolactam, an unwanted by-product.

The reductive amination is performed in the presence of a hydrogenation catalyst comprising ruthenium on

titanium oxide carrier as for example described in WO-A-9835938 or in US-A-5977356. Optionally, the catalyst contains at least one further group 8-11 metal or compounds thereof as for example described in WO-A-0014062. Of the further group 8-11 metal Co, Rh, Ir, Ni, Pd, Pt and Cu are preferred. The most preferred further group 8-11 metal is Rh and Ni.

A relatively small but catalytically effective amount of the catalyst is used in the step 3 of the present process. The amount of ruthenium (as metal) in the catalyst (metal plus carrier) is generally between 0.1 and 10 wt%. The amount of the group 8-11 metal (as metal) in the catalyst (metals plus carrier) is generally between 0.05 and 30 wt.%, preferably between 0.1 and 10 wt.% and more preferably between 0.1 and 5 wt.%. The molar ratio of ruthenium to the other metal is generally within the range from 100 : 1 to 1 : 10, preferably from 20 : 1 to 1 : 1. The mean particle size ($d_{50}$) of the catalyst is preferably between 10 and 100 μm, when the catalyst is present as a slurry in the reaction mixture or between 0.001 and 0.05 m, when the catalyst is present in a fixed bed. The BET surface area can be between 1 and 100 $m^2/g$. The BET surface area is preferably between 30 and 100 $m^2/g$. Preferably the titanium oxide catalyst carrier is used in its anatase form to reach such a high BET surface area of titanium oxide. The high BET surface area is advantageous because higher catalyst activity can be obtained.

The catalyst can be prepared by any of the processes as described in WO-A-0014062.

The molar ratio of ammonia and formyl-starting compound in the reductive amination step is preferably between about 3:1 and about 30:1, and more preferably between about 5:1 and about 20:1.

The temperature is preferably between about 40°C and about 200°C, and more preferably between about 80°C and about 160°C.

The process is preferably conducted under pressure. In general, the pressure is equal or greater than the resulting equilibrium pressure of the liquid reaction mixture employed. The pressure is preferably between 0.5 and 12 MPa.

The molar amount of hydrogen is at least equal to the molar quantity of formyl-starting compound. The molar ratio of hydrogen to the formyl-starting compound is preferably between about 1 to about 100.

The reductive amination can be performed batch wise or continuously. A large scale commercial process will preferably be performed continuously.

The reductive amination can be performed continuously in a fixed bed reactor in which the heterogeneous hydrogenation catalyst is present. An advantage of this reactor is that the reactants are easily separated from the hydrogenation catalyst. Another manner of performing the reductive amination is by way of one or more continuously operated well mixed contactors in series in which the hydrogenation catalyst is present as a slurry (slurry reactor). This manner of operation has the advantage that the concentration gradients and the heat of the reaction can be easily controlled. Examples of specific and suitable slurry reactors are one or multiple staged bubble columns or a gas lift-loop reactor or a continuously stirred tank reactor (CSTR). The slurry-hydrogenation catalyst can be separated from the reaction mixture by for example using hydrocyclones and/or by filtration, for example by cake- or cross-flow filtration.

The catalyst concentration can be suitably selected across a wide concentration range. In a fixed bed reactor the amount of catalyst per reactor volume will be high, while in a slurry-reactor this concentration will, in general be lower. In a continuously operated slurry reactor the weight fraction of catalyst (including the carrier) is typically between about 0.1 and about 30 weight % relative to the total reactor content.

In a subsequent step, which is optionally in the process according to the invention, the ε-caprolactam precursors present in the reaction mixture obtained in the reductive amination step are further reacted to ε-caprolactam. This reaction step, which is optionally but preferably carried out in the process of the invention, is hereinafter referred to as the cyclisation step.

Before feeding the reductive amination reaction mixture to the cyclisation step, ammonia, hydrogen, the heterogeneous hydrogenation catalyst and the alkanol (if present) are preferably separated from the reaction mixture obtained in the reductive amination. Hydrogen and ammonia can advantageously be separated from this reaction mixture by reducing the pressure and performing a gas/liquid separation. An example of such an operation is a flash operation performed in one or more steps at between about ambient pressure and about 1.5 MPa. Advantageously, the hydrogen and ammonia can be recycled to the reductive amination step. In a subsequent step, the alkanol (if present) can advantageously be separated as for example described in WO-A-9730973. It has been found advantageous to perform the cyclisation in the presence of less than 1 wt.% and more preferably less than 0.1 wt.% of alkanol because the presence of an alkanol during the cyclisation promotes the formation of the corresponding N-alkyl caprolactam, an unwanted by-product causing yield loss and/or making the purification more difficult. It is therefore favourable that these N-alkylated products do not form or that their formation is minimized in the cyclisation. Separating the alkanol from the reductive amination reaction mixture can be performed by suitable methods known to those skilled in the art, for example distillation or stripping, for example steam stripping. Preferably, the alkanol is removed by stripping the aqueous mixture with steam as described in WO-A-9730973. In a commercial large scale process the stripping preferably involves the continuous counter current contacting of the aqueous starting mixture with upflowing steam in a vertical positioned column, in which at the top a water/alkanol stream an at the bottom an alkanol-poor aqueous product stream is obtained. The steam stripping is preferably performed at a pressure between ambient pressure and 1 MPa,

and more preferably in the range of 0.1 and 0.3 MPa. Steam stripping is advantageous because the alkanols can be removed very effectively and because a convenient concentration of the ε-caprolactam precursors and ε-caprolactam in resulting aqueous mixture can be obtained, at which also stable storage of the product mixture is possible. The water/alkanol/ammonia mixture thus obtained can be recycled to the reductive amination step or can advantageously further subjected to a separation methods for separating ammonia and water. Separating the ammonia from the water/alkanol/ammonia mixture can be performed by suitable methods known to those skilled in the art, for example distillation. The ammonia thus separated is preferably recycled to the reductive amination reactor. In a subsequent step, the alkanol is preferably removed by distilling the water/alkanol mixture obtained in the ammonia distillation step. The thus separated alkanol can be advantageously partly or completely recycled to the carbonylation step (1) of the process according to the invention. In a continuous operated process, recycling of the thus separated alkanol to the carbonylation step results in that less fresh alkanol needs to be fed to the process of the invention.

The reaction mixture which is fed to the cyclisation step preferably contains between 10-50 wt.% 6-aminocaproic acid, between 10-50 wt.% 6-aminocaproamide, between 0-0.5 wt.% 6-aminocaproate ester, between 0-40 wt.% ε-caprolactam and between 0-15 wt.% oligomers of 6-aminocaproic acid and/or 6-aminocaproamide (the total amount of 6-aminocaproic acid, 6-aminocaproamide, 6-aminocaproate ester, ε-caprolactam and oligomers of 6-aminocaproic acid and/or 6-aminocaproamide being 100 wt.%). Optionally water can be removed from the product mixture by distillation, before it is fed to the cyclisation reactor. If desired, the starting compounds can even be fed to the reactor in a finely divided solid form.

The cyclisation is preferably performed as for example described in WO-A-9837063. Several methods for preparing caprolactam from caprolactam precursors have been described in the prior art. For example, US-A-4730040 describes a process in which 6-aminocaproic acid is converted to caprolactam in the liquid phase in the absence of an additional catalyst at a temperature of 300 °C and a pressure of up to 2 MPa. In such a process a liquid reaction mixture containing caprolactam, unconverted 6-aminocaproic acid and oligomers is obtained. WO-A-9837063 on the other hand describes a process in which 6-aminocaproic acid or derivatives thereof is contacted with steam in the absence of a catalyst at a temperature between 250 and 400 °C, preferably between 270 and 350 °C and a pressure of between 0.5 and 2 MPa, preferably below 1.5 MPa. In such a process, caprolactam is obtained in the gaseous phase, which phase does not consist substantial amounts of unconverted starting compounds and/or oligomers. The gaseous phase process as described in WO-A-9837063 has been found to be advantageous because the lower pressures can be used and the recovery of caprolactam is easier compared to the process as for example described in US-A-4730040.

The cyclisation is preferably carried out in a continuous mode. Part of the steam can be substituted with an inert stripping gas, for example nitrogen. The steam-feed ratio (in weight) is preferably between 1 and 20. This feed is the total weight of ε-caprolactam precursors and ε-caprolactam present in the starting mixture. This feed does not include any water which may be present. The cyclisation step is preferably performed as a continuous process in which steam is continuously fed to a reaction zone in which the starting compound is present. More preferably the starting compound or mixture of starting compounds is continuously or semi-continuously fed to the reactor zone, in which the feed has a temperature of between room temperature and the temperature of the reactor zone, preferably the temperature is between 50-280°C. The continuously operated cyclisation step can be practiced in a reactor apparatus which is provided with an inlet for the starting material, an outlet for the steam/ε-caprolactam product and means for supplying steam such that the steam is contacted with the starting material. The reactor is optionally equiped with a heating device and optionally with a mixing device. To this reactor the starting compound and the steam can be continuously fed. A possible reactor is a fluidized bed reactor containing inert particles in which the bed is kept fluidized by the steam. Another example of a reactor is a horizontal tube reactor having a rotating axis on which axis means for mixing and/or transport are present. Also means are present which prevent fouling of the interior vessel wall and which promote an optimal steam/substrate contact area for mass-transfer. Examples of suitable reactors are packed tower-type reactor, one or multiple staged bubble columns or a multi-tube reactor.

The gaseous product stream obtained in the cyclisation step will comprise caprolactam, steam, lights and heavies. Compounds having a higher boiling point than caprolactam are designated as heavies in this specification. Examples are 6-aminocaproic acid, 6-aminocaproamide, oligomers of these compounds, ε-caprolactam cyclic oligomers, N-substituted or C-substituted lactams and/or amides. Compounds having a lower boiling point than caprolactam are designated as lights in this specification. Examples are N-methyl-ε-caprolactam, hexanoic acid, 5-hexenoic acid, valeric acid and valeramide. Preferably, caprolactam is isolated from this gaseous reaction mixture by performing the following steps:

a) the product stream is fed to a partial condensation unit and split in a top stream comprising steam and a liquid bottom stream comprising ε-caprolactam, water, lights and heavies;
b) the bottom stream obtained in step a) is fed to a distillation column of which the top stream is mainly water and the bottom stream comprises ε-caprolactam, lights and heavies;

c) the bottom stream obtained in step b) is fed to a vacuum distillation column of which the top stream is mainly lights and the bottom stream comprises ε-caprolactam and heavies;

d) the bottom stream obtained in step c) is fed to a vacuum distillation column of which the top stream is the ε-caprolactam stream and the bottom stream is the heavies stream.

The heavies stream obtained in step d) can be disposed of (preferably after having removed caprolactam) or can be recycled to the cyclisation reactor. In case the heavies stream is recycled, a part of the heavies stream is advantageously purged and the rest is recycled to the cyclisation reactor.

The condensation of the product stream (step a)) is preferably performed at a temperature of 80-200°C, more preferably at a temperature of 120-190°C.

The distillation in step b) is for example performed at a temperature of 60-160°C, preferably at a temperature of 80-140°C.

The distillation in step c) and d) is preferably performed at a pressure lower than 10 kPa, more preferably at a pressure lower than 5 kPa. The temperature of the distillation in step c) and d) is preferably between 90°-170°C.

The ε-caprolactam stream resulting from the process according to the invention can be purified according to conventional techniques, for example extraction or crystallisation. Advantageously the caprolactam is purified by a crystallisation process as described in for example WO-A-9948867 and WO-A-0047557. The use of crystallisation results in ε-caprolactam with a higher purity.

The purification of ε-caprolactam by crystallisation can be conducted in the following steps:

e) the ε-caprolactam stream obtained in step d) is fed as a liquid ε-caprolactam stream into a crystallizer, in which conditions are set such that ε-caprolactam crystals and a mother liquid are formed,

f) the stream from the crystallizer is fed to a separator and split to purified ε-caprolactam and a mother liquid,

g) part of the mother liquid is recycled to the crystallizer.

Preferably, a part of the mother liquid is purged and the purge is recycled to the distillation column of step b) or to the distillation column of step c).

The crystallizer is operated such that crystallization of ε-caprolactam occurs through cooling. In the crystallizer relatively pure ε-caprolactam crystals are formed (solid phase) and a mother liquid, which comprises ε-caprolactam, impurities and optionally solvent (liquid or melt phase). The solid phase in the crystallizer can have a different appearance, depending on the way the crystallization is performed. The crystallization in the crystallizer can be performed for example either by cooling via a heat exchanging surface (suspension or layer crystallization) or by adiabatic cooling by evaporation of part of the contents of the crystallizer, for instance a solvent, under reduced pressure (crystallization in suspension). The method of crystallization induced by reduced pressure cooling is preferred, since no crystallization on inner cooling surfaces of the crystallizer occurs. In reduced pressure cooling the condensed vapour from the crystallizer may or may not be returned, totally or partially to the contents of the crystallizer. Preferably the crystallizer is operated by evaporating the solvent under reduced pressure.

Preferably solvent is present in the mixture in the crystallizer, although crystallization can also be conducted without solvent. Many solvents are suitable. Examples of suitable solvents are water, alkanes (like n-hexane, n-heptane, iso-octane, cyclohexane), alcohols (like methanol, ethanol, n-propanol, butanol), aromatic hydrocarbons (like benzene, toluene, o-xylene, m-xylene, p-xylene), ammonia, chlorinated hydrocarbons (like tetrachloromethane, chloroform or ethylchloride), ketones like acetone or methylethyl keton) and esters (like ethyl acetate). Preferably water and aromatic hydrocarbons are used as solvent, since these solvents give large crystals. Most preferred as solvent is water. The solvent will act as a freezing point depressor for the melt in the crystallizer.

The concentration of solvent in the melt in the crystallizer is dependent on the solvent, the amounts of impurities in the feed ε-caprolactam and the way the cooling in the crystallizer is performed. With the preferred solvent water and reduced pressure cooling the concentration of water in the melt is usually below 20 weight %, preferably 1-15 weight % and more preferred 2-10 weight %.

A solvent stream may be directly fed to the crystallizer and/or is mixed with the liquid crude ε-caprolactam feed stream prior to being fed to the crystallizer.

The temperature of the mixture in the crystallizer is dependent on the presence and concentration of solvent and impurities in the mixture, but at most 69°C, being the melting temperature of pure ε-caprolactam. Preferably the temperature of mixture in the crystallizer is 20-69°C, more preferable 35-67°C. A preferred mode of operation the crystallizer is described in *Chemical Engineering Sciences,* **2000,** 55, pages 3575-3584. The crystallizer can be operated in batch or in continuous mode. Preferably the crystallizer is operated in a continuous mode.

The separator of step f) may be any separator that is capable of separating crystals from the mother liquid, e.g. a filter working under forces like gravity, reduced pressure, increased pressure or a centrifuge. Various types of filters and centrifuges can be used. In these separators during or after separation washing of the crystals is possible

and preferred. The separator is for instance a horizontal vacuum belt filter. This type of solid-liquid separator has an excellent washing efficiency. Another example of a separator is a crystal washcolumn, in which the crystals are compacted into a packed bed which bed is transported with gravity, hydraulic pressure or a mechanical means. An example of a crystal washcolumn in which the crystal bed is transported with a mechanical means is a Niro screw-type wash column system as for example described in *European Chemical News,* 30 June - 6 July 1997, page 23. A crystal washcolumn has the advantage that an effective separation of the ε-caprolactam crystals from the mother liquid is achieved and simultaneously very effective washing of the crystals is performed. A more preferred crystal washcolumn is the so-called TNO-Thijssen hydraulic wash column as described in D. Verdoes, G.J. Arkenbout et al., "Improved procedures for separating crystals for the melt", *Applied Thermal Engineering,* **1997,** 17 (8-10), 879-888.

In the TNO-Thijssen hydraulic wash column, the purified ε-caprolactam crystals are removed from the crystal bed and subsequently molten by a heat exchanger. A part of the molten ε-caprolactam crystals is recycled to the crystal washcolumn as washing liquid. The ε-caprolactam washing liquid finally crystallizes on the surface of ε-caprolactam crystals present in the so-called washfront. This is advantageous because, with a minimum quantity of washing liquid, a very effective separation of the ε-caprolactam crystals from the mother liquid and simultaneously a high washing efficiency of the ε-caprolactam crystals is achieved. In the TNO-Thijssen hydraulic wash column the purified ε-caprolactam is obtained as a liquid melt.

Advantageously the purified ε-caprolactam from the separator is further purified in a second crystallization step followed by a second separation/washing step of the ε-caprolactam crystals. This second crystallization step may be performed in a similar way as the first crystallization step. The separation and effective washing of the ε-caprolactam crystals from the mother liquid can be performed in a solid-liquid separation equipment as described for separation/washing step. Preferably the stream leaving from the second crystallizer comprising ε-caprolactam crystals and a mother liquid, is fed to a centrifuge and split to a mother liquid and purified ε-caprolactam. The mother liquid is recycled to the second crystallizer. Preferably a part of the mother liquid is purged and the purge is recycled to the first crystallizer. If necessary additional crystallization steps and separation/washing steps are possible.

It has been found that the caprolactam obtainable with the process of the invention caprolactam is essentially free of contaminations that are produced by processes commonly used in practice. In the process according to the invention, the obtained caprolactam is as pure or even purer than the caprolactam obtained in processes commonly used in practice. It has been found that with the process of the invention a composition is obtained comprising (a) caprolactam and (b) less than 100 ppm 5-methyl-2-piperidinone and less than 10 ppm 4-ethyl-2-pyrrolidinone and/or 3-methyl-2-piperidinone.

The invention therefore also relates to such a composition.

The invention will be elucidated by the following examples, however these are not intended to limit the scope of the invention in any way.

EXAMPLE I

A solution containing 5.6 grams of a bidentate phosphite ligand with structure:

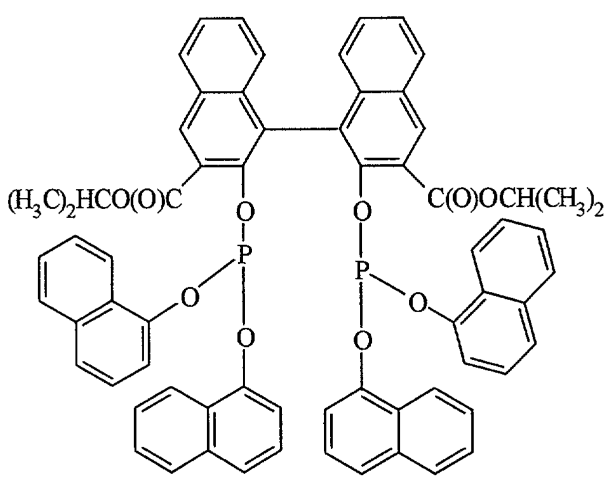

having a molweight of 1090 grams/mol, 1.10 grams of $RhAcAc(CO)_2$, 16 grams tri-ortho-tolyl phosphine and 576 grams

of m-xylene was prepared. A Hasteloy-B reactor with a nominal volume of 1 L was pressurized with CO and $H_2$ to 1 MPa ($CO/H_2$ = 1 (mol/mol)). The reactor was charged with 200 grams of the catalyst solution and 300 grams of freshly distilled methyl-3-pentenoate (M3P), heated to 95□C and left to for two hours while continuously feeding CO and $H_2$ in a 1:1 ratio at a flow rate of 30 Nl/hr each to the autoclave.

After these two hours a continuous feed to the autoclave of 90 grams/hr M3P and 80 grams/hr of catalyst solution was started originating from storage vessels containing these compounds. The catalyst solution feed was maintained until a total of 483 grams of solution was charged to the autoclave. The reactor pressure was subsequently reduced to 0.5 MPa. The level of liquid in the reactor was kept at a maximum of approximately 500 ml of liquid. Any excess liquid and any unreacted gasses will exit the reactor via a dip tube. This exit stream was let down to atmospheric pressure via a back pressure regulator and fed into a gas-liquid separator. The gas was -after passing through a condensor to remove condensables- vented. The liquid was collected in the bottom of the gas liquid separator from where it is fed through a control valve to a first rolled film evaporator operated at 90°C. In this evaporator most of the unreacted M3P, light by-products and a small part of the methyl formylvalerate (MFV) products were evaporated under vacuum (approximately 0.05 MPa). The liquid residue obtained in the first evaporation was passed through a column filled with an amount of 7 grams of a weakly basic Amberlist A21 (ion exchange) resin. From there it was pumped to a second rolled film evaporator. In this evaporator the remainder of the unreacted M3P and light byproducts and part of the methylformylvalerate product was evaporated.

The residue of the second evaporator was pumped back into the reactor thereby closing the loop. The temperature and the pressure of both evaporators were adjusted such that a constant liquid inventory of 1200 ml (if calculated back to the reactor) is maintained in the whole set-up as described above. Approximately 4 hours after starting the M3P feed all distillations and pumps were operating.

After 16 hours the set-up reached stable operating conditions. The Rh concentration in the reactor was 280 ppm. A small excess of ligand to rhodium was detectable by liquid chromatography. From that time on ligand was fed to the system by feeding 2 ml an hour of a solution of 2.9 grams of the bidentate phosphite ligand in 360 grams of m-xylene. The concentration of mono methyl adipate was <0.01 wt% at the end of 65 hours of operation. It was found that the conversion of M3P at constant M3P feed rate and hold up time in the reactor dropped during this period of 65 hours continuously from 80% to 75% at a selectivity to M5FV dropping from 82.5 to 81.5%.

After 65 hours 6 grams of the weak acid mono methyl adipate were charged to the system. From that time on until the conclusion of the experiment 100 hours later the conversion of M3P -and thus the activity of the remaining catalyst species- remained constant at 75%. The selectivity to M5FV increased from 81.5% to 82.5%. These results are summarized in Table 1. During the experiment no oxidation of the ligand was found to have occured. At the end of the experiment after 160 hours the tri-ortho-tolyl phosphine was found to be partly oxidized and the ligand concentration was 0.62 mgram/gram of reaction content.

Table 1

| reaction time (hours) | 40 | 70 | 165 |
|---|---|---|---|
| ligand/rhodium (mol/mol) | 1.05 | 1.10 | 1.20 |
| mono methyladipate (wt%) | <0.01 | 0.2 | 0.2 |
| conversion of M3P (%) | 79 | 75 | 75 |
| selectivity of methyl 5-formylvalerate (%) | 82.5 | 81.5 | 82.5 |

This example shows that when a continuous process is performed in the presence of a weak acid further deactivation of the catalyst is prevented.

EXAMPLE II

132 grams of 5 wt% ruthenium and 0.3 wt% nickel on titanium oxide (BET surface area 55 m2/g) were introduced in a mechanically stirred 1.5 liter Hastelloy-C reactor. After the addition of water, the catalyst was prereduced at 140 °C during 12 hours. Subsequently, an aqueous stream consisting of 25 wt% methyl-5-formylvalerate, 40 wt% ammonia, and 7 wt% methanol in water, was fed continuously to the reactor at a rate of 1095 grams/hour. The reactor was kept at a constant pressure of 4.0 MPa by a hydrogen stream of 7.5 grams per hour. The reaction was performed

at 140 °C. During 154 hours the effluent which continuously left the reactor was analyzed at regular intervals. A constant yield of desired products, i.e. ε-caprolactam and ε-caprolactam precursors, of 99.8% was obtained.

At the chosen reactor conditions approximately 95% methyl-groups of the methyl ester (methyl-6-aminocaproate M6AC) were 'hydrolyzed' and released as methanol. Since M6AC is a precursor for N-methyl caprolactam in the cyclization step this precursor was subsequently completely converted to ε-caprolactam and other ε-caprolactam precursors by flashing the reactor product at 0.5 MPa and subsequently feeding the flashed product mixture to a tube packed with inert glass pearls having a void volume of approximately 1 ltr. After further flashing, the remaining product mixture was continuously fed at a rate of 3.7 kg/hr to the top section of a distillation column having a diameter of 0.07 m and packed with 2 meter Sulzer BX packing.This column was operated at 0.1 MPa pressure, a bottom temperature of 102-105 °C, a top temperature of 60-70 °C and without any reflux (steam stripper). Thus ammonia and methanol were completely removed via the top. This is important because methanol is not allowed in the cyclization step (precursor for the N-methylcaprolactam formation.)This is also important because methanol and ammonia have to be recycled to the carbonylation step and to the reductive amination step respectively. The aqueous bottom product contained approximately 50 wt% ε-caprolactam and ε-caprolactam precursors. The product distribution of the organic part of this mixture was (wt%) 35 % of ε-caprolactam, 11 % of 6-aminocaproic acid, 40 % of 6-aminocaproic amide and 14 % of N6-oligomers.

EXAMPLE III

The product mixture of Example II was subsequently dewatered in a continuously operated oldershaw column with a diameter of 0.05 cm, 10 and 5 sieve-trays in top- and bottom-sections respectively. The feed rate was 1.1 kg/hr, the reflux-ratio was 0.3, and the column was operated at 150 mbar and a bottom temperature of 110 °C. The thus obtained product mixture contained 3.3 wt% of residual water and was fed continuously to a 500 ml autoclave having a turbine mixer. The autoclave was initially charged with 61 grams of the same organic feed mixture. After flushing with nitrogen and adjusting the pressure valve such that the pressure in the reactor was 1.2 Mpa, the temperature was raised to 300 °C. At the same time the continuous feed of the organic mixture started at a rate of 38 gr/hr and the steam at a rate of 222 g/hr. The ε-caprolactam content of the product phase was continuously analyzed. After approximately 10 hours a steady-state was reached at a ε-caprolactam concentation in the condensate of 12.9 wt%. The reactor content at steady state amounted to approximately 100 grams, present as oligomers of ε-caprolactam and 6-aminocaproic acid.

After 29 hours the feed rate was increased to 57 g/hr of the organic feed and 333 g/hr of steam. At that point of the continuous run the overall conversion of the ε-caprolactam precursors fed to the reactor was approximately 91 %.

After another 10 hours, the ε-caprolactam concentration in the condensed steam was 12.9 wt% indicating that steady-state was reached again. The reactor contents in the new steady-state conditions was approximately 140 grams. The reaction was continued for a total of 54 hrs. The overall conversion was 94.4 % at the end of the run. Subsequently the substrate feed was stopped and the run was continued in batch-mode at a steam-feed of 390 g/hr. After 7 hours the condensed steam contained approximately 120 gram of ε-caprolactam. The autoclave contained 7 grams of residue, corresponding to an overall conversion of 99.7 % of the organic feed. Overall 17700 gram of an aqueous product mixture was obtained which contained approximately 12.9 wt% ε-caprolactam.

EXAMPLE IV

From the crude ε-caprolactam product mixture obtained in Example III the bulk of water was evaporated using a rotary evaporator at a pressure of 10.5 kPa and a water bath temperature of 60 °C, yielding 3130 gram 72.8 wt% aqueous ε-caprolactam solution. The composition of this product was analyzed using HPLC and GC-MS methods. Excluding water, the product contained 98.0 wt% ε-caprolactam. Approximately 0.9 wt% of light impurities and 1.1 wt% of heavy impurities were present.

Subsequently residual water, lights and heavies were successively removed by batch distillation of the product in three separate distillation stages using a laboratory vigreux column.

During the first stage mainly water was removed at a column pressure that was gradually reduced from 8 to 0.2 kPa and the bottom temperature increased from 62 to 85 °C. In the second stage the lights fraction was distilled off at a column pressure of 0.3 kPa and a bottom temperature that gradually increased from 91 to 122 °C. At the end of this stage approximately 3 % of the ε-caprolactam was collected in the light fractions.

In the third stage 2150 gram (95.1 %) of the ε-caprolactam was distilled over the top at a pressure of 0.1 kPa and a bottom temperature gradually raising from 120 to 140 °C. At the end of this stage 68 grams of residue was collected. According to the mass-balance 1.7 % of the original amount of ε-caprolactam ended up in this heavies residue, corresponding to approximately 60 % of the total weight of this residue fraction.

Subsequently, the heavies residue was used as substrate in the following batch experiment:

A 500 ml autoclave having a turbine mixer was filled with the heavies residue. The autoclave was substantially flushed with nitrogen and a pressure valve was adjusted such that the pressure in the reactor was maintained at 1.2 Mpa. When the temperature reached 300 °C a steam flow was started of 300 g/hr. The steam containing ε-caprolactam leaving the reactor was condensed and analyzed. After 5 hours the condensed steam contained approximately 62 grams of ε-caprolactam, which corresponds to a practically complete conversion of ε-caprolactam precursors to ε-caprolactam.
The resulting 2 grams of residue did not yield any more caprolactam upon extended reaction.

EXAMPLE V

Crude ε-caprolactam obtained after only water removal of stage 1 in Example IV contained 6345 ppm of N-methyl caprolactam, 100 ppm of methyl-valerolactam and 78 ppm valeramide among other impurities. 73.6 grams of this distilled crude ε-caprolactam product was further purified by melt crystallization according to the following procedure.
Water was added to the crude caprolactam, to obtain a mixture containing 10 wt% water. The mixture was heated to 50 °C to obtain a homogeneous melt.
Subsequently the temperature was slowly reduced to 30 °C with a rate of approximately 10 °C per hour, while stirring mechanically. During the cooling down a caprolactam crystals slurry was formed. When the temperature had reached 30 °C the crystals were separated by means of filtration, and subsequently washed 2-3 times with a saturated aqueous solution of caprolactam.
33.7 grams of pure caprolactam crystals were obtained, containing 51 ppm of N-methyl caprolactam, 1 ppm methyl-valerolactam and 1 ppm of valeramide. Specifications were determined by following methods:
The E290 is 0.14, VB is 0.7 meq/kg. The E290 and VB values were measured according to the procedure described at the end of this experimental section. This single-step crystallization procedure resulted in purified product which meets the E290 specifications, and almost meets VB specifications for caprolactam obtained by Beckmann rearrangement.

Determination of the specifications was carried out in the following manner:

E290: Determination of the absorbance at a wavelength of 290 nm (E290) was carried out according to ISO method 7059, Caprolactam for industrial use - by determination of the absorbance of a 50 wt.% caprolactam solution in water at 290 nm, using a quartz cell with 4 cm path length.
Volatile Bases (VB): (cf. ISO method 7059, Caprolactam for industrial use - Determination of volatile bases content - Titrimetric method after distillation) A test sample of caprolactam was distilled in an alkaline medium. The volatile bases were liberated from the sample, taken up in 0.01 N hydrochloric acid, and determined by titration with 0.01 N sodium hydroxide solution.

$$VB = (((V_0 - V_1) \times 0.01)/\text{grams sample}) \times 1000 \text{ meq/kg}$$

where $V_0$ = volume, in milliliters, of the standard sodium hydroxide solution used in the blank test, and $V_1$ = volume, in milliliters, of the standard sodium hydroxide solution used in the determination.

EXAMPLE VI

The product of Example V was recrystallized by the same procedure. The product contains 2 ppm N-methyl caprolactam, 1 ppm of methyl-valerolactam and < 1 ppm of valeramide. E290 is 0.02, VB is < 0.4 meq/kg.
This example shows that by means of two crystallization steps pure caprolactam can be obtained which meets the VB specifications from caprolactam by Beckmann rearrangement.

EXAMPLE VII

Example V was repeated, with the exception that crude ε-caprolactam of Example IV was used after water, lights and heavies removal by a three stage distillation over a short vigreux column prior to the crystallization. 45.7 grams of distilled caprolactam, containing 2121 ppm of N-methyl caprolactam, 85 ppm of methyl-valerolactam and 69 ppm of valeramide among other impurities, was crystallized.
23.8 grams of pure caprolactam crystals were obtained, containing 39 ppm of N-methyl caprolactam, 1 ppm of methyl-valerolactam and 2 ppm of valeramide. The addition of the distillation prior to crystallization results in a further

improvement of the E290 to 0.05 and VB to 0.41 meq/kg compared to a single step crystallization procedure, and results in a product which meets the E290 and VB specifications.

Example VIII

0.16 grams of 5 wt.% ruthenium on titania was introduced in a 160 ml autoclave. The catalyst was reduced at 150 °C at 5.0 MPa hydrogen during one hour. After addition of 100 ml ammonia, the temperature was brought to 92 °C and the pressure to 7.0 MPa. Subsequently 3.1 grams methyl-5-formylvalerate and 1.5 grams methanol were added. After 30 minutes reaction the total yield of ε-caprolactam and ε-caprolactam precursors was 86%.

Example IX

0.16 grams of 5 wt.% ruthenium on alumina was introduced in a 160 ml autoclave. The catalyst was reduced at 150 °C at 5.0 MPa hydrogen during one hour. After addition of 100 ml ammonia, the temperature was brought to 92 °C and the pressure to 7.0 MPa. Subsequently 3.0 grams methyl-5-formylvalerate and 1.4 grams methanol were added. After 30 minutes reaction the total yield of ε-caprolactam and ε-caprolactam precursors was 34%.

Example X

0.32 grams of 5 wt.% ruthenium on titania was introduced in a 160 ml autoclave. The catalyst was reduced at 150 °C at 5.0 MPa hydrogen during one hour. After addition of 100 ml ammonia, the temperature was brought to 92 °C and the pressure to 7.0 MPa. Subsequently 4.3 grams methyl-5-formylvalerate were added. After 10 minutes reaction the total yield of ε-caprolactam and ε-caprolactam precursors was 32%.

Example XI

0.32 grams of 5 wt.% ruthenium on alumina was introduced in a 160 ml autoclave. The catalyst was reduced at 150 °C at 5.0 MPa hydrogen during one hour. After addition of 100 ml ammonia, the temperature was brought to 92 °C and the pressure to 7.0 MPa. Subsequently 4.4 grams methyl-5-formylvalerate were added. After 10 minutes reaction the total yield of ε-caprolactam and ε-caprolactam precursors was 19%.

**Claims**

**1.** A process for the preparation of ε-caprolactam, **characterized in that**, ε-caprolactam is prepared starting from butadiene, carbon monoxide, hydrogen and ammonia and the process comprises:

1. carbonylating butadiene in the presence of an alkanol and a catalyst comprising palladium, a multidentate phosphine ligand and an acidic co-catalyst to produce alkyl-4-, alkyl-3- and alkyl-2-pentenoate,
1'. optionally isomerising the alkyl-3- and/or alkyl-2-pentenoate into alkyl-4-pentenoate,
2. hydroformylating the alkyl-4-, alkyl-3- and alkyl-2-pentenoate in the presence of a catalyst comprising rhodium and an organic phosphorous containing ligand to produce alkyl-5-formylvalerate,
3. reductively aminating alkyl-5-formylvalerate in the presence of a hydrogenation catalyst comprising ruthenium on a carrier catalyst to produce ε-caprolactam and ε-caprolactam precursors,
4. optionally converting ε-caprolactam precursors at elevated temperature into ε-caprolactam.

**2.** Process according to claim 1, **characterized in that**, the multidentate phosphine ligand is a bidentate phosphine ligand selected from one or more symmetric or asymmetric [3,3,1] or [4,2,1]isomers of 1,2-P,P'bis(1,5-dimethyl, 9-phosphabicyclononyl)ethane, symmetric or asymmetric [3,3,1] or [4,2,1] isomers of 1,3-P,P'bis(1,5-dimethyl, 9-phosphabicyclononyl)propane, symmetric or asymmetric [3,3,1] or [4,2,1] isomers of 1,2-P,P'bis (1,5-dimethyl 9-phosphabicyclononyl)propane.

**3.** Process according to any of claims 1-2, **characterized in that**, step (1') is omitted.

**4.** Process according to any one of claims 1-3, **characterized in that**, the organic phosphorous containing ligand is a multidentate phosphite ligand with the following general formula

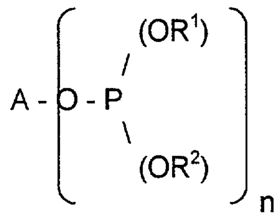

where $R^1$ and $R^2$ are the same or different monovalent aromatic organic groups which are not connected to each other in any way other than via the phosphorous atom P and where A is an n-valent group or atom and where n is an integer of at least 2 and where the respective [-O-P($OR^1$)($OR^2$)] group may be the same group or different groups and where the phosphite forms a chelate-type complex with rhodium.

**5.** Process according to claim 4, **characterized in that**, the bridging group A is a 2,2'-dihydroxyl-1,1'-binaphtalene bridging group with the following structure

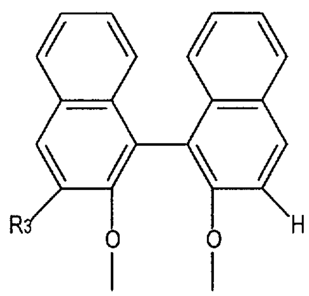

or

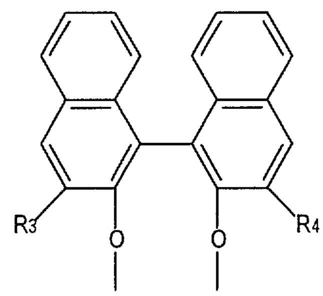

which $R^3$ and $R^4$ are substituents other than hydrogen.

**6.** Process according to claim 5, chararacterized in that, $R^3$ and $R^4$ are individually a carboalkoxyl or a carboaryloxy group, $-CO_2R$, in which R is a $C_1$-$C_8$ alkyl group.

**7.** Process according to any one of claims 4-6, **characterized in that**, the multidentate phosphite ligand is a bidentate phosphite ligand (n=2).

**8.** Process according to any of claims 1-7, **characterized in that**, the hydroformylation is performed in the presence of mono methyl adipate.

**9.** Process according to any one of claims 1-8, **characterized in that**, the hydroformylation is performed in the presence of trio-tolyl)phosphine).

**10.** Process according to any one of claims 1-9, **characterized in that**, the reductive amination is performed in the presence of a ruthenium on titanium oxide carrier hydrogenation catalyst.

**11.** Process according to any one of claims 1-10, **characterized in that**, the reductive amination is performed in a water/corresponding alkanol mixture as solvent.

**12.** Process according to any one of claims 1-11, **characterized in that**, the reaction mixture obtained in the reductive amination step (3) is contacted with steam in the absence of a catalyst at a temperature between between 270 and 350 °C and a pressure below 1.5 MPa.

**13.** Process according to claim 12, **characterized in that**, alkanol is separated from the mixture fed to the cyclisation step such that less than 1 wt.% of alkanol is present in the mixture fed to the cyclisation step (4).

**14.** Process according to claim 13, **characterized in that**, the thus separated alkanol is partly recycled to the carbonylation step (1).

**15.** Process according to any one of claims 12-14, **characterized in that**, caprolactam is isolated form the gaseous reaction mixture obtained in the cyclisation step by performing the following steps

a) the product stream is fed to a partial condensation unit and split in a top stream comprising steam and a liquid bottom stream comprising ε-caprolactam, water, lights and heavies;
b) the bottom stream obtained in step a) is fed to a distillation column of which the top stream is mainly water and the bottom stream comprises ε-caprolactam, lights and heavies;
c) the bottom stream obtained in step b) is fed to a vacuum distillation column of which the top stream is mainly lights and the bottom stream comprises ε-caprolactam and heavies;
d) the bottom stream obtained in step c) is fed to a vacuum distillation column of which the top stream is the ε-caprolactam stream and the bottom stream is the heavies stream.

**16.** Process according to claim 15, **characterized in that**, the ε-caprolactam stream obtained in step d) is purified in the following steps

e) the ε-caprolactam stream obtained in step d) is fed as a liquid ε-caprolactam stream into a crystallizer, in which conditions are set such that ε-caprolactam crystals and a mother liquid are formed,
f) the stream from the crystallizer is fed to a separator and split to purified ε-caprolactam and a mother liquid,
g) part of the mother liquid is recycled to the crystallizer.

**17.** ε-caprolactam obtainable by the process according to claim 16.

**18.** Composition comprising (a) ε-caprolactam and (b) 1-100 ppm 5-methyl-2-piperidinone and less than 10 ppm 4-ethyl-2-pyrrolidinone and/or 3-methyl-2-piperidinone.

European Patent Office

# EUROPEAN SEARCH REPORT

Application Number
EP 01 20 1356

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,Y | WO 00 56695 A (JAGER WILLEM WABE ;DRENT EIT (NL); SHELL INT RESEARCH (NL)) 28 September 2000 (2000-09-28) see whole document, especially pages 5,6 and claim 10. | 1-18 | C07D201/08 |
| D,Y | US 5 495 041 A (SIELCKEN OTTO E ET AL) 27 February 1996 (1996-02-27) see especially column 3, lines 5-15 | 1-18 | |
| D,Y | WO 97 33854 A (DSM NV ;DU PONT (US)) 18 September 1997 (1997-09-18) * the whole document * | 1-18 | |
| D,Y | WO 00 14062 A (DSM NV ;PESTMAN ROBERT (NL); LIESHOUT LAMBERTUS HUBERTUS WI (NL);) 16 March 2000 (2000-03-16) * the whole document * | 1-18 | |
| D,A | EP 1 028 109 A (DSM NV) 16 August 2000 (2000-08-16) | 1-18 | TECHNICAL FIELDS SEARCHED (Int.Cl.7) |
| X | * the whole document * | 17,18 | C07D |
| D,Y | WO 98 37063 A (GUIT RUDOLF PHILIPPUS MARIA ;BUIJS WIM (NL); DSM NV (NL); AGTERBER) 27 August 1998 (1998-08-27) | 1-18 | |
| X | * the whole document * | 17,18 | |
| X | US 5 962 680 A (EISENSCHMID THOMAS CARL ET AL) 5 October 1999 (1999-10-05) * the whole document * | 1-18 | |

The present search report has been drawn up for all claims

| Place of search | Date of completion of the search | Examiner |
|---|---|---|
| MUNICH | 13 September 2001 | Scruton-Evans, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 01 20 1356

This annex lists the patent family members relating to the patent documents cited in the above-mentioned European search report.
The members are as contained in the European Patent Office EDP file on
The European Patent Office is in no way liable for these particulars which are merely given for the purpose of information.

13-09-2001

| Patent document cited in search report | Publication date | Patent family member(s) | Publication date |
|---|---|---|---|
| WO 0056695 A | 28-09-2000 | AU 3290700 A | 09-10-2000 |
| US 5495041 A | 27-02-1996 | CN 1141286 A | 29-01-1997 |
| | | EP 0728733 A | 28-08-1996 |
| | | JP 8245509 A | 24-09-1996 |
| WO 9733854 A | 18-09-1997 | AU 2045197 A | 01-10-1997 |
| | | CA 2249026 A | 18-09-1997 |
| | | DE 69703035 D | 12-10-2000 |
| | | DE 69703035 T | 12-04-2001 |
| | | EP 0888274 A | 07-01-1999 |
| | | ES 2152085 T | 16-01-2001 |
| | | JP 2000506525 T | 30-05-2000 |
| | | US 6018081 A | 25-01-2000 |
| | | US 5874641 A | 23-02-1999 |
| WO 0014062 A | 16-03-2000 | EP 0984002 A | 08-03-2000 |
| | | AU 5535699 A | 27-03-2000 |
| | | EP 1109781 A | 27-06-2001 |
| EP 1028109 A | 16-08-2000 | AU 2580100 A | 29-08-2000 |
| | | WO 0047557 A | 17-08-2000 |
| WO 9837063 A | 27-08-1998 | EP 0860431 A | 26-08-1998 |
| | | AU 6006198 A | 09-09-1998 |
| | | CN 1246846 T | 08-03-2000 |
| | | DE 69801154 D | 23-08-2001 |
| | | EP 0968184 A | 05-01-2000 |
| | | US 6194572 B | 27-02-2001 |
| US 5962680 A | 05-10-1999 | CN 1297433 T | 30-05-2001 |
| | | CN 1260779 T | 19-07-2000 |
| | | EP 0975591 A | 02-02-2000 |
| | | EP 0975590 A | 02-02-2000 |
| | | PL 336212 A | 05-06-2000 |
| | | PL 336266 A | 19-06-2000 |
| | | WO 9846565 A | 22-10-1998 |
| | | WO 9846564 A | 22-10-1998 |
| | | US 5925754 A | 20-07-1999 |

EPO FORM P0459

For more details about this annex : see Official Journal of the European Patent Office, No. 12/82